# EUROPÄISCHE PATENTSCHRIFT

(11) **EP 1 795 125 B1**
(45) Veröffentlichungstag und Bekanntmachung des Hinweises auf die Patenterteilung: **08.08.2012**
(21) Anmeldenummer: 06123656.8
(22) Anmeldetag: 08.11.2006
(51) Int. Cl.: G08B 21/04, A61B 5/11, A61B 5/00

(54) **Notfallüberwachungsvorrichtung für ältere oder behinderte Personen**
Device for monitoring elderly or handicapped persons in an emergency situation
Dispositif de surveillance de personnes agées ou handicapées en cas d'urgence

(30) Priorität: 29.11.2005 DE 102005056757
(43) Veröffentlichungstag der Anmeldung: 13.06.2007
(73) Patentinhaber: ROBERT BOSCH GMBH, 70442 Stuttgart (DE)
(72) Erfinder: Thuersam, Markus, 71263 Weil Der Stadt (DE); Tuermer, Joerg, 85560 Ebersberg (DE)

(56) Entgegenhaltungen:
- WO-A-2004/093023
- WO-A-2005/008914
- US-A1- 2002 060 630
- US-A1- 2002 116 080

## Beschreibung

### Stand der Technik

Notfallüberwachungssysteme dienen zur Erhöhung der passiven Sicherheit beispielsweise von älteren oder behinderten Personen im täglichen Leben. Durch diese Systeme erfolgt eine ständige Überwachung dieser Personen ohne diese jedoch in ihrer persönlichen Freiheit durch die ständige Anwesenheit von Pflegepersonal zu beschränken und unterstützen somit ein selbstbestimmtes Leben im Alter oder bei Behinderungen.

Üblicherweise umfassen die Notfallüberwachungssysteme einen Sender, der ständig von der zu überwachenden Person z.B. in Form eines Armbandes getragen wird und an dem ein Notfallknopf angeordnet ist, der im Notfall betätigt wird. Der Sender übermittelt dann ein Signal an eine Empfangsstation, die das Notfallsignal in geeigneter Weise an eine Überwachung weitergibt.

Die Druckschrift EP 1 128 359 A1 offenbart eine Sturzdetektionsvorrichtung und ein entsprechendes Softwareprodukt, welche automatisch den Sturz einer Person detektieren. Es wird eine Sensorvorrichtung offenbart, die von der zu überwachenden Person während der Überwachung ständig getragen werden muss, und die einen Stosssensor und einen Richtungssensor aufweist. Auf Basis der Auswertung der Signale dieser beiden Sensoren wird gegebenenfalls ein Sturz von der Sensorvorrichtung detektiert und ein Notrufsignal an eine Überwachung gesendet.

Die Fa. Cairos Technologies AG, Deutschland bietet ein Notfallüberwachungssystem an, welches mehrere Empfangseinrichtungen und eine an der zu überwachenden Person in Form eines Armbands angebrachte Sendervorrichtung umfasst. Die Sendervorrichtung weist Sensoren zur Messung verschiedener persönlicher Daten wie z.B. Blutdruck und Puls auf. Sobald die persönlichen Daten einen kritischen Wert passieren, wird ein Alarm an ein Rettungsteam gesendet. Das Rettungsteam erhält als Einsatzinformation Informationen über den Zustand des Patienten in Form der persönlichen Daten sowie dessen aktuelle Position.

Die Druckschrift WO 2004/093023 A2, die wohl den nächstkommenden Stand der Technik bildet, betrifft eine Artikelerfassungs- und -verfolgungsvorrichtung sowie ein entsprechende Verfahren. Bei einigen Ausführungsformen ist die offenbarte Vorrichtung zur räumlichen Ortung von mindestens einem an dem Körper einer Person befestigbaren Sender ausgebildet. Der Sender weist einen Beschleunigungssensor auf, welcher die Detektion eines Sturzes auf Basis der Auswertung der Messsignale des Beschleunigungssensors erlaubt.

### Offenbarung der Erfindung

Die erfindungsgemäße Notfallüberwachungsvorrichtung ist für die Verwendung durch beispielsweise ältere oder behinderte Personen ausgebildet und wird bevorzugt als Erweiterung für Hausnotrufsysteme oder sonstige Rufsysteme eingesetzt. Ein Notfall liegt insbesondere vor, wenn die zu überwachende Person in einen hilfsbedürftigen Zustand gerät und/oder einen Unfall erleidet.

Die Notfallüberwachungsvorrichtung umfasst eine Ortungsvorrichtung, die ausgebildet ist, um eine Ortung von mindestens einem Sender durchzuführen, der an dem Körper, z.B. an der Kleidung, der zu überwachenden Person befestigbar ist. Zweckmässigerweise wird mindestens eine zweidimensionale Ortung realisiert; so dass die Position des Senders beispielsweise relativ zu einem bekannten Grundriss eines Hauses oder einer Wohnung feststellbar ist. Es ist jedoch bevorzugt, dass eine räumliche Ortung durchgeführt wird, insbesondere so, dass dreidimensionale Ortsinformationen des mindestens einen Senders bestimmbar sind und/oder bestimmt werden. Insbesondere wird durch die Ortungsvorrichtung die genaue Position des Senders in einem zu überwachenden Bereich, wie z.B. einem Zimmer, einer Wohnung oder einem Haus bestimmt.

Eine Auswerteeinrichtung der Notfallüberwachungsvorrichtung ist ausgebildet, um die ermittelten Ortungsinformationen der Ortungsvorrichtung aufzunehmen und daraus ein Bewegungsabbild des mindestens einen Senders zu bilden. Vorzugsweise wird an Hand einer Vielzahl registrierter Einzelpositionen des mindestens einen Senders mindestens eine Trajektorie, also mindestens eine Ort-Zeit-Kurve, gebildet. Vorzugsweise ist die Ortungsvorrichtung und/oder die Auswerteeinrichtung stationär und der mindestens eine Sender als mobile Einheit ausgebildet.

Erfindungsgemäß ist die Auswerteeinrichtung programmtechnisch und/oder schaltungstechnisch so realisiert, dass durch Auswertung des Bewegungsabbilds ein Notfall oder einer Notfallsituation der Person detektierbar ist und/oder detektiert wird.

Gegenüber dem bekannten Stand der Technik schlägt die Erfindung einen neuen Weg ein, indem sie zur Detektion eines Notfalls nicht Sensoren verwendet, die direkt an der zu überwachenden Person angeordnet sind, sondern die Bewegung der Person von außen beobachtet und die aktuelle Situation auf Basis der Beobachtung bewertet.

Durch diesen neuartigen Ansatz zur Detektion eines Notfalls ist es möglich weitaus mehr Informationen zur Bewertung einer Situation heranzuziehen. Beispielsweise ist nicht nur eine abrupte Beschleunigung, die z.B. durch einen Aufschlag der Person auf dem Boden hervorgerufen ist, detektierbar, sondern zusätzlich die Richtung der Beschleunigung. Es ist somit möglich, zwischen einem Anstoßen der Person, also einer horizontalen Beschleunigung, und einem Sturz der Person, also einer vertikalen Beschleunigung, zu unterscheiden. Weitere Auswertungsmöglichkeiten ergeben sich dadurch, dass eine absolute Höhenänderung des mindestens einen Senders und somit der Person detektierbar ist und die wichtigste Kenngröße eines Sturzes- nämlich eine abrupte Höhenänderung - durch einfache Auswertealgorithmen erkennbar ist.

Ein weiterer Vorteil ergibt sich dadurch, dass energiekonsumierende Berechnungen zur Detektion eines Notfalls in stationäre (z.B. netzversorgte) Teile der Notfallüberwachungsvorrichtung verlegt sind. Der an dem Körper der zu überwachenden Person anzubringende Sender wird von dieser Aufgabe entlastet mit der Folge, dass die Lebensdauer der sendereigenen Energieversorgung stark verlängert wird. Dadurch ist es möglich, dass auch Personen die Notfallüberwachungsvorrichtung über einen längeren Zeitraum nutzen können, die nicht mehr in der Lage sind, selbstständig die mobile Einheit, also den mindestens einen Sender, in eine Ladevorrichtung einzulegen.

Weitere Vorteile ergeben sich aus den Merkmalen der abhängigen Ansprüche sowie der nachfolgenden Beschreibung.

Bevorzugt ist die Notfallüberwachungsvorrichtung über ein Übertragungsnetzwerk, beispielsweise dem öffentlichen Telefonnetz, an eine Empfangseinheit zur Entgegennahme von Notrufen angeschlossen. Die Empfangseinheit wird beispielsweise bei dem Hausarzt, einer Pflegestation oder dem Krankenhaus aufgestellt, so dass bei einem detektierten Notfall umgehend Hilfsmaßnahmen eingeleitet werden können.

Bei einer vorteilhaften Weiterbildung ist die Auswerteeinrichtung zur Erkennung eines Sturzes der Person ausgebildet. Hierzu wird das Bewegungsabbild des mindestens einen Senders und somit der zu überwachenden Person ermittelt und ausgewertet, wobei bei einem Vorliegen charakteristischer Merkmale in dem Bewegungsabbild ein Sturz detektiert wird. Vorzugsweise wird nach Detektion des Sturzes eine Notfallmeldung über das Übertragungsnetzwerk an die Empfangseinheit gesendet. Die Detektion des Sturzes erfolgt bevorzugt durch Auswertung der Ort-Zeit-Kurve, beispielsweise durch einen Mustervergleich mit einer gespeicherten " Referenz-" Fallkurve, und/oder durch Auswertung der Geschwindigkeit - Zeit - Kurve, beispielsweise durch einen Vergleich mit einem abgespeicherten Grenzwert einer Maximalgeschwindigkeit, und/oder durch Auswertung der Beschleunigung - Zeit - Kurve, beispielsweise durch einen Vergleich mit einem abgespeicherten Grenzwert einer Maximalbeschleunigung. Insbesondere wird bei der Auswertung die Bewegungsrichtung des mindestens einen Senders berücksichtigt, wobei beispielsweise vertikale Bewegungen des Senders eher als Sturz interpretiert werden als horizontale Bewegungen.

Bei einer vorteilhaften Weiterbildung ist die Ortungsvorrichtung als Kreuzortungsvorrichtung ausgebildet und weist mindestens zwei Empfangsstationen auf, wobei die mindestens zwei Empfangsstationen miteinander insbesondere schaltungstechnisch, z.B. über ein Netzwerk, und/oder kabellos, z.B. über Funk, verkoppelt sind und/oder gemeinsam einen Verbund bilden. Die Empfangsstationen können beispielsweise in üblichen Raumsprechstellen oder Hausnotrufteilnehmerstationen integriert sein. Vorzugsweise ist die Auswerteeinrichtung in einer Empfangsstation angeordnet, die insbesondere als Mutterstation ausgebildet ist.

Bei einer vorteilhaften Weiterbildung ist die Ortungsvorrichtung zur parallelen und/oder alternierenden Ortung von mindestens zwei Sendern ausgebildet, so dass entweder gleichzeitig und/oder abwechselnd Ortungsinformationen von den mindestens zwei Sendern von der Ortungsvorrichtung empfangen werden. Die Auswerteeinrichtung ist ausgebildet, um durch Auswertung der relativen Lage der Sender zueinander und/oder der globalen Lage der Sender Zustandsinformationen über die zu überwachende Person zu ermitteln.

Eine optionale Weiterbildung ist gegeben, wenn im Betrieb mindestens zwei Sender an der zu überwachenden Person angeordnet sind. Vorzugsweise werden die mindestens zwei Sender mit einem großen Abstand an der zu überwachenden Person positioniert, insbesondere an verschiedenen Extremitäten der Person. Beispielsweise wird jeweils ein Sender an den Handgelenken der Person befestigt, so dass durch die Verbindungslinie der zwei Sender eine Körperachse senkrecht zur Längserstreckung der Person gebildet ist. Alternativ ist ein Sender am Handgelenk und ein anderer Sender am Bein oder am Fußgelenk angeordnet, so dass durch die Verbindungslinie eine Körperachse definiert ist, die diagonal zur Längserstreckung der Person ausgerichtet ist. Bei einer weiteren Alternative sind die Sender entlang der Längserstreckung der Person angeordnet. Ferner ist eine Kombination der genannten Alternativen möglich. Bei dieser Weiterbildung ist die Auswerteeinrichtung ausgebildet, um durch die Auswertung der relativen und/oder absoluten Lage der Sender Zustandsinformationen hinsichtlich der Körperhaltung der Person (z. B. Sitzen, Liegen, Laufen) und/oder der absoluten Lage (z. B. Sturz) der Person zu ermitteln. Insbesondere kann bei einem möglichen Sturz die Endposition des Körpers eindeutig erkannt werden.

Eine andere optionale Weiterbildung ist gegeben, wenn mindestens ein Sender an der zu überwachenden Person angeordnet ist und mindestens ein weiterer Sender an einem mobilen Gegenstand, wie z.B. einer Sitzgelegenheit, einem Rollator oder einem Rollstuhl, befestigt ist. Die Auswerteeinrichtung ist ausgebildet, um aus der Relativlage und/oder Absolutlage der mindestens zwei Sender Zustandsinformationen zu ermitteln, wie z.B. ob die Person in einem Rollstuhl sitzt oder einen Rollator bedient. Die genannten Zustandsinformationen können vorteilhaft zur Detektion eines Notfalls genutzt werden.

Vorzugsweise umfasst die Notfallüberwachungsvorrichtung mindestens einen der an dem Körper einer Person und/oder an mobilen Gegenständen befestigbaren Sender, wobei der Sender vorzugsweise eine eigene Energieversorgung aufweist und/oder durch ein in dem Raum ausgestrahltes elektromagnetisches Feld versorgt wird und/oder versorgbar ist. Der Sender ist vorzugsweise zur zyklischen Emission eines kodierten Signals ausgebildet. Die Kodierung erlaubt die Unterscheidung verschiedener Sender durch die Ortungsvorrichtung. Insbesondere wird das Signal mehrmals pro Sekunde emittiert. Optional ist vorgesehen, dass das Signal als Antwort auf einen externen Sendeimpuls ausgelöst wird und/oder auslösbar ist.

Bei einer weiteren vorteilhaften Ausbildung ist die Notfallüberwachungsvorrichtung realisiert, so dass das Bewegungsabbild in Abhängigkeit der absoluten Lage oder Position des mindestens einen Senders in dem zu überwachenden Umfeld bewertet wird. Vorzugsweise sind in der Notfallüberwachungsvorrichtung Informationen über das zu überwachende Umfeld der zu überwachenden Person hinterlegt. Derartige Informationen sind z.B. als Notfallbedingungen ausgebildet, die je nach Umfeldabschnitt unterschiedlich formuliert sein können. Beispielsweise kann in den Notfallbedingungen für einen Umfeldabschnitt "Küche" hinterlegt sein, dass ein Hinlegen der zu überwachenden Person auf den Boden als Notfall detektiert wird und z.B. ein sofortiger Alarm ausgelöst wird. Dagegen sind beispielsweise die Notfallbedingungen für einen Umfeldabschnitt "Schlafzimmer" so definiert, dass ein Hinlegen der zu überwachenden Person nicht als Notfall detektiert wird.

Optional sind die Notfallbedingungen in Abhängigkeit der Zeit formulierbar und/oder formuliert, so dass beispielweise das Hinlegen der zu überwachenden Person in dem Umfeldabschnitt "Schlafzimmer" vor 11 Uhr vormittags nicht, jedoch nach 11 Uhr als Notfall detektiert wird.

Insbesondere können Umfeldabschnitte festgelegt sein, in denen ein bestimmter Personenzustand, wie z.B. Sitzen oder Liegen, zur Detektion eines Notfalls führen, wohingegen in anderen Umfeldabschnitten oder Bereichen dieser Personenzustand als normal detektiert wird. Optional wird ein Notfall detektiert, sobald die zu überwachende Person einen vorher definierten Bereich betritt oder verlässt.

Diese Ausbildungen bergen den Vorteil, dass bei der Detektion eines Notfalls durch die Ergänzung von Ortsinformationen der zu überwachenden Person, die Bewegungsabbilder unterschiedlich bewertet werden und ein Notfall zuverlässiger und schneller erkannt wird.

In einer Weiterbildung ist die Auswerteeinrichtung programmtechnisch und/oder schaltungstechnisch ausgebildet, so dass die Detektion eines Notfalls unter Berücksichtigung der Ortsinformationen über die absolute Position der zu überwachenden Person, der Ortsinformation eines an einem mobilen Gegenstand angebrachten Senders, Zusatzinformationen, insbesondere hinsichtlich der Körperhaltung, oder einer beliebigen Kombination dieser Informationen erfolgt. Insbesondere die Berücksichtigung der mobilen Gegenstände hat den Vorteil, dass deren aktuelle Lage bei der Detektion eines Notfalls und/oder der Auswahl von Notfallbedingungen mitberücksichtigt werden kann.

Vorzugsweise verwendet das Notfallüberwachungssystem das nachfolgend beschriebene Verfahren und/oder Computerprogramm.

Die Erfindung betrifft weiterhin ein Verfahren zur Detektion eines Notfalls von einer mit wenigstens einem Sender ausgestatteten Person, insbesondere unter Verwendung der erfindungsgemäßen Notfallüberwachungsvorrichtung. Hierbei wird in einem ersten Schritt der wenigstens eine Sender geortet, in einem weiteren Schritt ein Bewegungsabbild des Senders und somit insbesondere der zu überwachenden Person erzeugt und in einem weiteren Schritt auf Basis des Bewegungsabbilds ein Notfall der zu überwachenden Person detektiert.

Bei einer Weiterbildung des Verfahrens wird die zu überwachende Person mit mindestens zwei Sendern ausgestattet, die vorzugsweise entweder parallel und/oder alternierend und insbesondere räumlich geortet werden. Aus den Ortungsinformationen der mindestens zwei Sender wird ein gemeinsames Bewegungsabbild erzeugt, welches Zustandsinformationen über die Ausrichtung von mindestens einer Körperachse der zu überwachenden Person enthält. Insbesondere wird über die Zustandsinformationen eine Körperhaltung wie z.B. Sitzen, Liegen, Stehen detektiert.

Bei einer weiteren vorteilhaften Ausgestaltung wird durch die Ortungsvorrichtung die genaue Position der Sender und somit der zu überwachenden Person registriert. Diese genaue Position wird anhand eines Abbilds der möglichen Aufenthaltsorte der zu überwachenden Person ausgewertet und der Aufenthaltsort der zu überwachenden Person bestimmt. Dem Abbild der möglichen Aufenthaltsorte sind ortsabhängig weitere Informationen oder Notfallbedingungen zugeordnet, die zur Detektion eines Notfalls verwendet werden. Diese Informationen oder Notfallbedingungen betreffen zulässige und nicht-zulässige Bereiche, zulässige und nicht-zulässige Körperhaltungen und/oder zulässige und nicht-zulässige Bewegungsmuster, Geschwindigkeitsmuster und/oder Beschleunigungsmuster des Bewegungsabbilds in bestimmten Bereichen. Die Ausdrücke zulässig und nicht-zulässig sind so zu verstehen, dass das Betreten eines nicht-zulässigen Bereichs oder eine nicht-zulässige Körperhaltung usw. auf einen Notfall hinweist und/oder zur Detektion eines Notfalls führt.

Ergänzend wird ein weiterer Sender an mobilen Gegenständen angebracht und die Ortungsinformationen des weiteren Senders bei der Detektion eines Notfalls berücksichtigt. Diese Ausgestaltung erlaubt, die momentane Situation der zu überwachenden Person besser einzuschätzen. Beispielsweise wird ein weiterer Sender in einen Treppenlift eingebaut. Ohne diese Maßnahme könnte die Benutzung des Treppenlifts durch die zu überwachende Person zu einer Fehldetektion eines Treppensturzes führen, da das Bewegungsabbild der Person im Bereich eines Treppenhauses eine für die sonstige Mobilität der zu überwachenden Person zu große Bewegungsgeschwindigkeit aufweist. Sobald aber registriert wird, dass die zu überwachende Person mit dem Treppenlift das Treppenhaus passiert, kann eine derartige Fehldetektion auf einfache Weise vermieden werden.

Die beschriebenen alternativen der Notfallüberwachungsvorrichtung und des Verfahrens können in beliebiger Kombination verwendet werden.

Die Erfindung betrifft auch ein Computerprogramm mit den Merkmalen des Anspruchs 10, welches Programmcode-Mittel zur Durchführung aller Schritte des erfindungsgemäßen Verfahrens und optional zur Durchführung der Schritte der Weiterbildungen des Verfahrens aufweist. Der Computer weist dabei eine beliebige Hardwarearchitektur auf und/oder ist beispielsweise als PC, embedded system, Mikrocontroller, DSP oder ähnlichem ausgebildet. Vorzugsweise wird das Computerprogramm auf der Ortungsvorrichtung und/oder der Auswerteeinrichtung der erfindungsgemäßen Notfallüberwachungsvorrichtung ausgeführt.

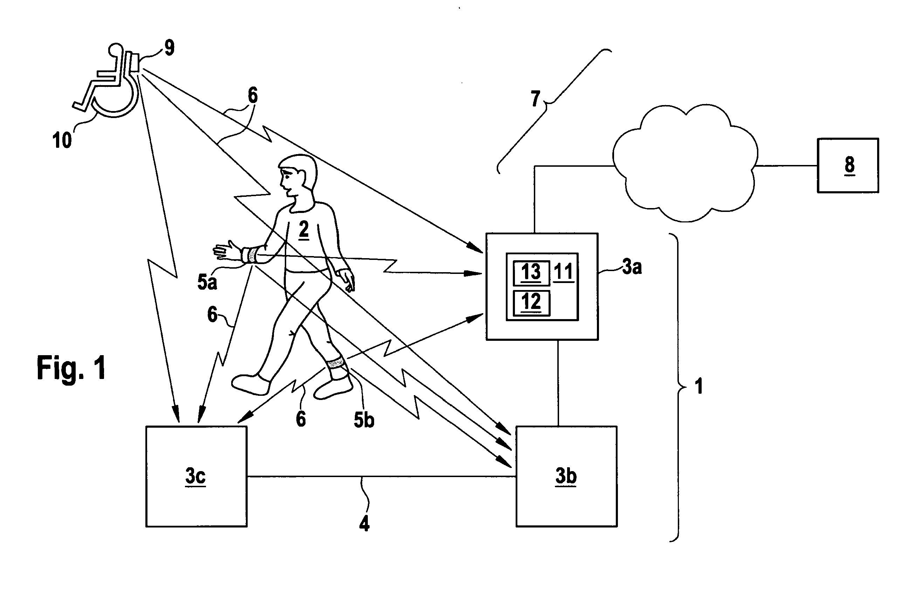

Weitere Einzelheiten, Merkmale, Merkmalskombinationen, Vorteile und Wirkungen auf der Basis der Erfindung ergeben sich aus der nachfolgenden Beschreibung eines bevorzugten Ausführungsbeispiels der Erfindung und der Zeichnung, wobei die Figur 1 in schematischer Darstellung das Ausführungsbeispiel einer erfindungsgemäßen Notfallüberwachungsvorrichtung zeigt.

Die Figur 1 zeigt eine Notfallüberwachungsvorrichtung 1 als ein Ausführungsbeispiel der Erfindung in schematischer Blockdarstellung mit der eine Person 2 überwacht wird.

Die Notfallüberwachungsvorrichtung 1 umfasst drei Empfangsstationen 3 a, b und c, die über ein Netzwerk 4 miteinander verbunden sind. An dem Körper der zu überwachenden Person 2 sind Sender 5 a und b als mobile Einheiten befestigt. Der Sender 5 a ist an einem Handgelenk und der Sender 5 b an einem Fußgelenk der Person 2 angeordnet. Die drei Empfangsstationen 3 a, b und c sind ausgebildet, um die genaue Position der Sender 5 a und b mittels einer Kreuzortung zu erfassen. Dabei werden von den Sendern 5 a und b Sendesignale 6 ausgesendet, die von den Empfangsstationen 3 a, b und c empfangen werden. Bei einer möglichen Ausgestaltung der Notfallüberwachungsvorrichtung 1 senden die Sender 5 a und b ein zyklisches Sendesignal 6 aus, so dass über die Zeitdifferenz zwischen Aussendung des Sendesignal 6 und dessen Empfang eine Entfernung zwischen den Sendern 5 a und b und den Empfangsstationen 3 a, b und c ermittelt werden kann. Durch den Empfang der Sendesignale 6 durch die an verschiedenen Orten platzierten Empfangsstationen 3 a, b und c wird die genaue Position mittels Kreuzortung ermittelt. Alternativ werden durch die Empfangsstationen 3 a, b und c ein Empfangswinkel und eine Entfernung bestimmt und durch bekannte Algorithmen auf die genaue Position der Sender 5 a und b geschlossen. Vorzugsweise weist jeder Sender 5 a und b ein unterschiedlich kodiertes Sendesignal 6 aus, so dass die Sender 5 a und b von der Empfangsstationen 3 a, b und c unterschieden werden können. Alternativ kann die Laufzeitmessung auch durch einen Signalrundweg und/oder ein Polling, also einen aktiven Sendeaufruf, durch die Empfangsstation an die Sender oder andersrum erfolgen. Die Häufigkeit der Laufzeitmessung kann situationsabhängig angepasst werden, z.B. wenn eine starke Bewegung der Sender 5a, b durch die Sender selbst, insbesondere über einen integrierten Bewegungssensor, oder durch die Empfangsstation 3a, b registriert wird.

Die Empfangsstation 3 a ist als Mutterstation ausgebildet und weist eine Auswerteeinheit 11, in der ein Computerprogramm 12 abläuft, sowie einen Speicher 13 auf, in dem Notfallbedingungen und/oder Informationen über die zu überwachende Umgebung hinterlegt sind.

Die Notfallüberwachungsvorrichtung 1 ist über ein weiteres Netzwerk 7, welches beispielsweise als Telefonnetz, Internet oder Funkverbindung ausgebildet ist, mit einer Empfangseinheit 8 verbunden, die bei einem Hausarzt, einem Rettungsteam oder einer Pflegerstation aufgestellt ist.

Ergänzend zu den Sendern 5 a und b ist ein weiterer Sender 9 an einem Rollstuhl 10 angeordnet, wobei die Sendesignale 6 des Senders 9 ebenfalls von der Empfangsstationen 3 a, b und c analog zu den Sendern 5 a und b empfangen und ausgewertet werden.

Bei dem Betrieb der Notfallüberwachungsvorrichtung 1 werden in einem vorbereitenden Schritt Notfallbedingungen zur Detektion eines Notfalls in dem Speicher 13 abgelegt. Optional werden Informationen über die zu überwachende Umgebung der Person 2 hinterlegt. Die Notfallbedingungen können zeitabhängig, ortsabhängig und/oder von anderen Bedingungen, wie z.B. dem Abstand der Person 2 zu einem mobilen Gegenstand wie dem Rollstuhl 10, formuliert sein.

Im Überwachungsbetrieb werden von den Sendern 5 a, b und 9 ständig Sendesignale 6 ausgegeben, die von den Empfangsstationen 3 a, b und c als Ortungsinformationen empfangen werden. Die Ortungsinformationen werden über das Netzwerk 4 zu der Auswerteeinheit 11 geleitet und dort durch Ablauf des Computerprogramms 12 weiterverarbeitet.

Aus den Ortungsinformationen wird ein Bewegungsabbild der Person 2 erstellt. Das Bewegungsabbild umfasst eine Trajektorie des Patienten 2 und optional die zeitlichen Ableitungen der Trajektorie. Diese Trajektorie bzw. deren zeitliche Ableitungen werden zur Detektion eines Notfalls z.B. mit hinterlegten Bewegungsmustern verglichen.

Durch die relative Lage der Sender 5 a und b zueinander kann eine Körperhaltung der Person 2 ermittelt werden, beispielsweise sind die Sender 5 a und b in Sitzhaltung enger beieinander als in Stehhaltung.

Durch die Berücksichtigung der absoluten Lage der Sender 5 a und b werden weitere Rückschlüsse auf den momentanen Zustand der Person 2 gezogen: Liegen beispielsweise die Sender 5 a und b in einer horizontalen Ebene und weisen einen Abstand größer als 50 Prozent der Körperhöhe der Person 2 auf, so kann auf eine liegende Haltung der Person 2 geschlossen werden. Sind zudem in dem Speicher 13 Informationen über die zu überwachende Umgebung hinterlegt, so können weitere Rückschlüsse über den Zustand der Person gezogen werden, wie nachfolgend erläutert wird. Ist die absolute Lage der Sender 5 a und b beispielsweise in der Höhe eines Fußbodens detektiert worden, so kann daraus geschlossen werden, dass die Person 2 gestürzt ist und sich liegend auf dem Fußboden befindet. Ist die absolute Lage der Sender 5 a und b in einem Bereich detektiert worden, in dem sich gemäß der hinterlegten Daten ein Bett befindet, so wird davon ausgegangen, dass sich die Person 2 auf dem Bett befindet und kein Notfall vorliegt. Des Weiteren können die Positionen von mobilen Gegenständen wie dem Rollstuhl 10 berücksichtigt werden.

Die Auswerteeinheit 11 sammelt die ermittelten Informationen über die Position und den Zustand der Person 2, das Bewegungsabbild, die Körperhaltung und über die Position von mobilen Gegenständen und wertet diese unter Berücksichtigung von orts- und/oder zeitabhängigen Notfallbedingungen zur Detektion eines Notfalls aus. Bei der Auswertung wird beispielsweise eine Wahrscheinlichkeitsfunktion, ein neuronales Netz, Fuzzy Logic oder insbesondere adaptive Filter verwendet. Als Ergebnis der Auswertung wird das Vorliegen oder das Nicht-Vorliegen eines Notfalls detektiert. Falls ein Notfall detektiert wird, wird über das zweite Netzwerk 7 ein Notruf an die Empfangseinheit 8 gesendet, der neben einer Ortsinformation über die Person 2 optional ergänzende Informationen über den Zustand oder die Körperhaltung der Person 2 enthält.

## Patentansprüche

1. Notfallüberwachungsvorrichtung (1) für ältere oder behinderte Personen (2) mit einer als Kreuzortungsvorrichtung mit drei Empfangsstationen (3 a, b, c) ausgebildeten Ortungsvonichtung (3 a, b, c) zur räumlichen Ortung von mindestens einem an dem Körper einer Person (2) befestigbaren Senders (5a, b) und mit einer Auswerteeinrichtung (11) zur Aufnahme der dreidimensionalen Ortungsinformationen der Ortungsvorrichtung (3a, b, c) und zur Bildung eines Bewegungsabbilds des mindestens einen Senders (5 a, b) aus den dreidimensionalen Ortungsinformationen, wobei die Auswerteeinrichtung (11) programmtechnisch und/oder schaltungstechnisch ausgebildet ist, einen Sturz der Person (2) zu detektieren,
**dadurch gekennzeichnet, dass**
der Sturz der Person (2) auf Basis des Bewegungsabbilds detektiert wird, indem die Beschleunigung - Zeit -Kurve des Senders ausgewertet wird, wobei zwischen einer horizontalen Beschleunigung und einer vertikalen Beschleunigung unterschieden wird.

2. Notfallüberwachungsvorrichtung (1) nach Anspruch 1, **dadurch gekennzeichnet, dass** die Ortungsvorrichtung (3 a, b, c) zur parallelen und/oder alternierenden Ortung von mindestens zwei Sendern (5a, b, 9) ausgebildet ist und die Auswerteeinrichtung (11) programmtechnisch und/oder schaltungstechnisch durch Auswertung der relativen Lage der Sender (5a, b, 9) zueinander und/oder der absoluten Lage der Sender (5a, b, 9) zur Detektion von Zustandsinformationen der Person (2) ausgebildet ist.

3. Notfallüberwachungsvorrichtung (1) nach Anspruch 1 oder 2, **dadurch gekennzeichnet, dass** die Auswerteeinrichtung (11) programmtechnisch und/oder schaltungstechnisch ausgebildet ist, um in Abhängigkeit von einer absoluten Lage des mindestens einen Senders (5a, b, 9) unterschiedliche Notfallbedingungen zur Detektion eines Notfalls auszuwählen.

4. Verfahren zur Detektion eines Notfalls von einer mit wenigstens einem Sender ausgestatteten Person, wobei der wenigstens eine Sender geortet wird, wobei aus den Ortungsinformationen ein Bewegungsabbild erzeugt wird und wobei ein Notfall auf Basis des Bewegungsabbilds detektiert wird, **dadurch gekennzeichnet, dass** die Detektion des Notfalls durch eine Notfallüberwachungsvorrichtung (1) gemäß der vorhergehenden Ansprüche erfolgt.

5. Verfahren nach Anspruch 4, wobei die Ortungsinformationen von mindestens zwei an der Person angeordneten und voneinander beabstandeten Sendern zur Erzeugung des Bewegungsabbilds verwendet werden und wobei das Bewegungsabbild Zustandsinformationen über die Ausrichtung von mindestens einer Körperachse der Person umfasst.

6. Verfahren nach Anspruch 4 oder 5, wobei zur Detektion eines Notfalls verschiedene anhand der Ortungsinformationen des mindestens eines Senders auszuwählende Notfallbedingungen definiert sind und/oder definierbar sind.

7. Verfahren nach einem der Ansprüche 4 bis 6, wobei die Ortungsinformationen eines weiteren an einem mobilen Gegenstands angebrachten Senders bei der Detektion des Notfalls berücksichtigt werden.

8. Computerprogramm (12) mit Programmcode-Mitteln, um alle Schritte von jedem beliebigen der Ansprüche 4 bis 7 durchzuführen, wenn das Programm auf einem Computer ausgeführt wird.

## Claims

1. Emergency monitoring apparatus (1) for elderly or handicapped people (2) having a locating apparatus (3a, b, c), in the form of a cross-locating apparatus with three receiving stations (3a, b, c), for physically locating at least one transmitter (5a, b) which can be attached to the body of a person (2) and having an evaluation device (11) for picking up the three-dimensional locating information from the locating apparatus (3a, b, c) and for forming a movement map for the at least one transmitter (5a, b) from the three-dimensional locating information, wherein the evaluation device (11) is designed, by means of programming and/or circuitry, to detect a fall by the person (2),
**characterized in that**
the fall by the person (2) is detected on the basis of the movement map by virtue of the acceleration/time curve of the transmitter being evaluated, wherein a distinction is drawn between a horizontal acceleration and a vertical acceleration.

2. Emergency monitoring apparatus (1) according to Claim 1, **characterized in that** the locating apparatus (3a, b, c) is designed to locate at least two transmitters (5a, b, 9) in parallel and/or alternately, and the evaluation device (11) is designed, by means of programming and/or circuitry, to evaluate the relative position of the transmitters (5a, b, 9) with respect to one another and/or the absolute position of the transmitters (5a, b, 9) in order to detect state information for the person (2).

3. Emergency monitoring apparatus (1) according to Claim 1 or 2, **characterized in that** the evaluation device (11) is designed, by means of programming and/or circuitry, to take an absolute position for the at least one transmitter (5a, b, 9) as a basis for selecting different emergency conditions for detecting an emergency.

4. Method for detecting an emergency for a person equipped with at least one transmitter, wherein the at least one transmitter is located, wherein a movement map is generated from the locating information and wherein an emergency is detected on the basis of the movement map, **characterized in that** the emergency is detected by an emergency monitoring apparatus (1) according to the preceding claims.

5. Method according to Claim 4, wherein the locating information is used by at least two transmitters, which are arranged on the person and are at a distance from one another, for generating the movement map and wherein the movement map comprises state information about the orientation of at least one body axis of the person.

6. Method according to Claim 4 or 5, wherein various emergency conditions that can be selected using the locating information from the at least one transmitter are defined and/or can be defined for the purpose of detecting an emergency.

7. Method according to one of Claims 4 to 6, wherein the locating information from a further transmitter fitted to a mobile article is taken into account for detecting the emergency.

8. Computer program (12) having program code means in order to perform all the steps of any one of Claims 4 to 7 when the program is executed on a computer.

## Revendications

1. Dispositif de surveillance des situations d'urgence (1) pour personnes âgées ou handicapées (2), comprenant un dispositif de localisation (3a, b, c) configuré sous forme de dispositif de localisation en croix avec trois stations réceptrices (3a, b, c) pour la localisation dans l'espace d'au moins un émetteur (5a, b) pouvant être fixé sur le corps d'une personne (2) et comprenant un dispositif d'interprétation (11) pour enregistrer les informations de localisation tridimensionnelles du dispositif de localisation (3a, b, c) et pour former une représentation du mouvement de l'au moins un émetteur (5a, b) à partir des informations de localisation tridimensionnelles, le dispositif d'interprétation (11) étant configuré par technique de programmation et/ou par technique de connexion pour détecter une chute de la personne (2), **caractérisé en ce que**
la chute de la personne (2) est détectée en se basant sur la représentation du mouvement en interprétant la courbe accélération/temps de l'émetteur, la différence étant faite entre une accélération horizontale et une accélération verticale.

2. Dispositif de surveillance des situations d'urgence (1) selon la revendication 1, **caractérisé en ce que** le dispositif de localisation (3a, b, c) est configuré pour la localisation en parallèle et/ou en alternance d'au moins deux émetteurs (5a, b, 9) et le dispositif d'interprétation (11) est configuré par technique de programmation et/ou par technique de connexion pour la détection d'informations d'état de la personne (2) par interprétation de la position relative des émetteurs (5a, b, 9) les uns par rapport aux autres et/ou de la position absolue des émetteurs (5a, b, 9).

3. Dispositif de surveillance des situations d'urgence (1) selon la revendication 1 ou 2, **caractérisé en ce que** le dispositif d'interprétation (11) est configuré par technique de programmation et/ou par technique de connexion pour sélectionner différentes conditions de situation d'urgence en fonction d'une position absolue de l'au moins un émetteur (5a, b, 9) en vue de la détection d'une situation d'urgence.

4. Procédé de détection d'une situation d'urgence d'une personne équipée d'au moins un émetteur, l'au moins un émetteur étant localisé, une représentation du mouvement étant formée à partir des informations de localisation et une situation d'urgence étant détectée en se basant sur la représentation du mouvement, **caractérisé en ce que** la détection de la situation d'urgence est effectuée par un dispositif de surveillance des situations d'urgence (1) selon les revendications précédentes.

5. Procédé selon la revendication 4, les informations de localisation d'au moins deux émetteurs disposés sur la personne et espacés l'un de l'autre étant utilisées pour générer la représentation du mouvement et la représentation du mouvement comprenant des informations d'état sur l'orientation d'au moins un axe du corps de la personne.

6. Procédé selon la revendication 4 ou 5, différentes conditions de situation d'urgence à sélectionner au moyen des informations de localisation de l'au moins un émetteur étant définies et/ou pouvant être définies en vue de la détection d'une situation d'urgence.

7. Procédé selon l'une des revendications 4 à 6, les informations de localisation d'un autre émetteur monté sur un objet mobile étant prises en compte lors de la détection de la situation d'urgence.

8. Programme informatique (12) comprenant des moyens de code de programme pour exécuter toutes les étapes de n'importe laquelle des revendications 4 à 7, lorsque le programme est exécuté sur un ordinateur.
